# EUROPEAN PATENT SPECIFICATION

(11) **EP 2 430 041 B1**
(45) Date of publication and mention of the grant of the patent: **31.10.2018**
(21) Application number: 09840273.8
(22) Date of filing: 27.05.2009
(51) Int. Cl.: C07K 14/37

(54) **CANCER CELL BINDING RECOMBINANT LECTINS WITH ANTITUMOR ACTIVITY AND METHOD OF PREPARATION**
AN KREBSZELLEN BINDENDE REKOMBINANTE LECTINE MIT ANTITUMORWIRKUNG UND HERSTELLUNGSVERFAHREN
LECTINES RECOMBINANTES SE LIANT À DES CELLULES CANCÉREUSES DOTÉES D'UNE ACTIVITÉ ANTITUMORALE ET PROCÉDÉ DE PRÉPARATION

(30) Priority: 18.02.2009 IN 350MU2009
(43) Date of publication of application: 21.03.2012
(60) Divisional application: 18187607.9
(73) Proprietor: Unichem Laboratories Limited, Mumbai - 100102, Maharashtra (IN); Karnatak University, Dharwad 580 003 (IN)
(72) Inventor: SWAMY, Bale Murugi, Dharwad 580003 (IN); INAMDAR, Shashikala Ramchandra, Dharwad 580003 (IN); VENKAT, Hemalatha, Mumbai 400026 (IN); CHACHADI, Vishwanath Basavaraj, Dharwad 580003 (IN); NAGRE, Nagaraja Narayan, Dharwad 580003 (IN); RAMADOSS, Candadai Seshadri, Mumbai 400026 (IN)
(74) Representative: J A Kemp
(86) International application number: PCT/IN2009/000306
(87) International publication number: WO 2010/095143

(56) References cited:
- MODY RUSTOM ET AL: "Use of Lectins as Diagnostic and Therapeutic Tools for Cancer", JOURNAL OF PHARMACOLOGICAL AND TOXICOLOGICAL METHODS, vol. 33, no. 1, 1995, pages 1-10, XP002610172, ISSN: 1056-8719
- WANG ET AL: "Lectins from mushrooms", MYCOLOGICAL RESEARCH, ELSEVIER, GB, vol. 102, no. 8, 1 August 1998 (1998-08-01), pages 897-906, XP022451362, ISSN: 0953-7562, DOI: DOI:10.1017/S0953756298006200
- SATHISHA G J ET AL: "X-ray sequence ambiguities of Sclerotium rolfsii lectin resolved by mass spectrometry", AMINO ACIDS ; THE FORUM FOR AMINO ACID AND PROTEIN RESEARCH, SPRINGER-VERLAG, VI, vol. 35, no. 2, 28 December 2007 (2007-12-28), pages 309-320, XP019634527, ISSN: 1438-2199
- WU ALBERT M ET AL: "Carbohydrate specificity of a lectin isolated from the fungus Sclerotium rolfsii", LIFE SCIENCES, vol. 69, no. 17, 14 September 2001 (2001-09-14), pages 2039-2050, XP002610173, ISSN: 0024-3205
- SHARON N ET AL: "Lectins--proteins with a sweet tooth: functions in cell recognition.", ESSAYS IN BIOCHEMISTRY 1995 LNKD- PUBMED:8822149, vol. 30, 1995, pages 59-75, XP9141288, ISSN: 0071-1365

## Description

### FIELD OF INVENTION

The invention relates to the field of carbohydrate binding proteins, and more specifically to a protein which binds specifically to particular oligosaccharides associated with particular medical significance. The invention specifically relates to lectins, more particularly to recombinant fungal lectins having ability to recognize glycans of clinical importance and their antiproliferative effect on cancer cells finding applications in cancer diagnosis, therapy and research.

### BACKGROUND OF INVENTION

Lectins are proteins, or glycoproteins that agglutinate erythrocytes of some or all blood groups in vitro. They are important group of bioactive proteins found in most organisms. Lectins are used as tools for diagnostic and therapeutic purpose in health care areas. Lectins are also used in the purification of glycoproteins, oligosaccharide analysis and in cell selection processes. Lectins can bind reversibly with monosaccharides or the sugar moiety of, glycoproteins or glycolipids or polysaccharides.

Changes in carbohydrate structures on the cell surfaces are a hallmark during cancer. Such changes results in exposure of tumor associated carbohydrate structures. Amongst them, is the increased occurrence of Thomsen-Friedenreich antigen, a disaccharide Galβ1→3GalNac-*O*-Ser/Thr. In normal cells, this antigen is masked by certain sugars, sialic acid or sulphates. Some of the lectins have ability to specifically recognize and bind to these altered structures and exerts various physiological effects such as apoptosis, cytotoxicity, proliferative/antiproliferative activity, metastasis, inhibition of cell adhesion, etc., Hence such lectins are currently being considered for use as cancer diagnostic and therapeutic agents and also for anti tumor activity.

Several lectins have been isolated and characterized from variety of sources. They however differ in their physico-chemical properties such as molecular size and sugar specificities. There is also characterization of a few recombinant proteins. The recombinant mushroom lectin from *Marasmius oreades* has a molecular size of 33 kDa and shows high affinity for Galα1, 3Gal and Galα1, 3Galβ1, 4GlcNAc. (R.P.Kruger et al., J.Biol.Chem., 277, 15002-15005, 2002; H.C.Winter et al. J.Biol.Chem ,277, 14996-15001, 2002., I.J. Goldstein, et al. US Patent :6958321)

Peanut lectin that shows affinity to Thomsen-Friedenreich antigen has been cloned (V.Sharma and A.Surolia, Gene (Amst), 148, 299-304, 1994) and subsequently some mutants of this protein with a subunit molecular mass of ∼28kDa has also been expressed in *E.coli* (V.Sharma, M.Vijayan and A.Surolia, J. Biol. Chem., 271, 21200-21213,1996) with some difference in its preference for sugar specificity. A much smaller lectin of molecular mass of 11.73 kDa with high binding affinity to L-fucose from *Pseudomonas aeruginosa* has also been expressed in *E.coli* as a Yellow fluorescent Protein-lectin fusion (BioTechniques, 41, 327-332, 2006).

A mushroom lectin (ABL) from *Agaricus bisporus,* which has antiproliferative activity on human colon cancer cells and its curative effect in psoriasis is reported by Jonathan M. Rhodes; US patent # 5607679.

*Sclerotium rolfsii* lectin (SRL) purified from the sclerotial bodies of the phytopathogenic fungus *Sclerotium rolfsii* exhibit complex carbohydrate specificity and exhibit specificity towards TF antigen. TF antigen is an oncofetal antigen, a disaccharide Galβ1, 3GaNAc α ser/thr and is known to express on cell surfaces in different cancers. SRL apart from binding to TF antigen, it also bind to sialylated and sulfated disaccharide. The crystal structure of this lectin and its complete amino acid sequence is determined (Demetres 2007 and Satisha 2008). Binding studies indicated that SRL binds to different human cancer tissues and also to cultured cells. Further binding of SRL to cultured human leukemic cells and colon cells results in their antiproliferation and in colon cells effect is shown to be mediated by inducing apoptosis. However the purified native lectin has solubility problems for its use in various applications, hence there is need of forms of this lectin with improved stability for practical applications.

Lectins thus have enormous potential in the area of medical assays. The instant application proceeds to provide certain lectins with novel characteristics so as to be useful in the medical field and improved properties.

### SUMMARY OF THE INVENTION

The present invention discloses recombinant lectins having specificity to certain sugar chains uniquely found on certain cancer cells. The present invention further provides method for preparing two recombinant lectins (designated as UC-SS/CSR-1803 and UC-SS/CSR-1805 or simply Rec-2 and Rec-3 respectively) expressed in *E.coli.* The method comprises the synthesis of lectin genes whose sequences are derived by deliberate modifications made to the original amino acid sequence of a lectin from *Sclerotium rolfsii* (Sathisha et.al., Amino Acids; 2008, 35, 309-320), a soil borne phytopathogenic fungus, and its cloning in *E.coli.* The recombinant lectins expressed as soluble proteins and are purified by ion exchange and gel filtration chromatographic techniques.

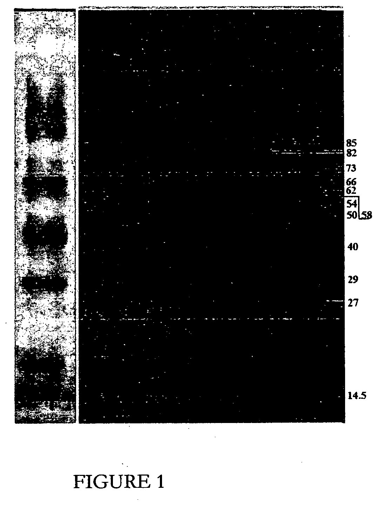

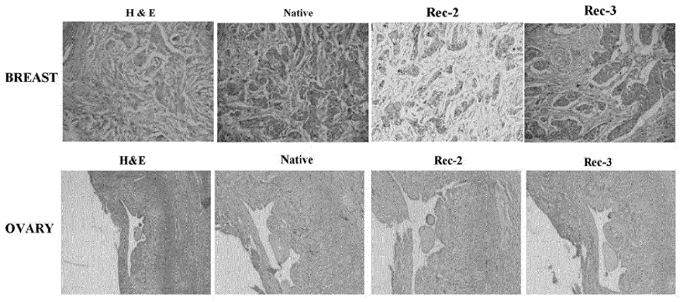

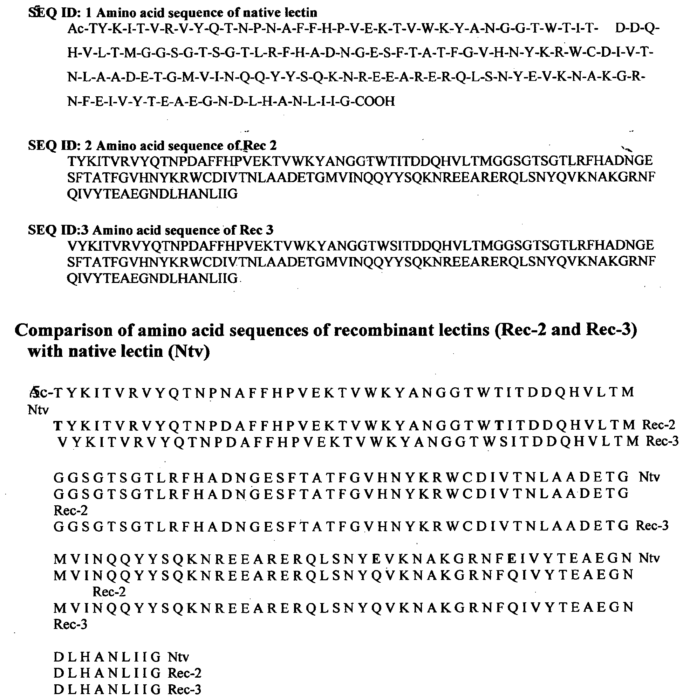

The present invention specifically provides two recombinant lectins (Rec-2 and Rec-3) having approximate subunit masses (Mᵣ 16.1 kDa), which are specific to certain O-glycosidically linked glycans occurring in glycoproteins and on cell surfaces, but does not show specificity to N-linked glycans.. The property of recognizing O-glycans but not N-glycans and showing no specificity to human blood groups makes them different from the other recombinant lectin disclosed in copending patent application 30/MUM/2008. It is also found that the recombinant lectins Rec-2 & Rec-3 does not bind to fetuin but exhibit very strong affinity for asialofetuin, there by making it different from that of said recombinant lectin (patent application 30/MUM/2008) and also the native lectin. By virtue of this recognition property they bind to certain cell surface molecules expressed during malignancy (Fig. 1) and thus bind to human Breast cancer, colon cancer, ovarian cancer tissues (Fig .2) and also cultured cells and leukemic cell lines (table 1). Apart from binding to cultured human cancer cells, recombinant lectins exert *in vitro* antiproliferative activity on the cells (Table 2) contemplating their diagnostic and therapeutic properties.

The invention discloses the DNA sequences for **Rec-2** and **Rec-3** coding for 141 amino acids long lectins in each case similar to the native lectin (*S. rolfsii* lectin), but with few deliberate modifications synthesized chemically and cloned into *E. coli* (SEQ ID 1.1 and SEQ ID 2.1). The cloned lectins were expressed after induction with the inducer IPTG. The expressed protein appeared as soluble and in active form in each case. The recombinant lectins were purified to essential homogeneity by resorting to anion exchange and molecular sieve chromatography. The mass spectrometric and SDS PAGE estimate of molecular weights show a value of approximate 16.1 kDa. The isoelectric focusing experiment shows the PI value of 6.614 whereas the native lectin has 6.219, the property markedly helped in improving the solubility and stability of recombinant proteins. Improved solubility, , stability and the binding to desialylated TF antigen of the recombinant lectins have greater implications of their applications in various fields as reagents apart from ease of production.

Also disclosed herein is a method for preparing a recombinant lectin expressed in a host cell such as *E.coli or yeast.* This method comprises the synthesis of a lectin gene based on amino acid sequence derived from the MALDI MS/MS of the *Sclerotium rolfsii* lectin (SEQ ID.1), a soil borne phytopathogenic fungus, and its cloning in the host cell.

In order to obtain desired properties the amino acid sequence of the native lectin was modified deliberately and recombinant lectins were obtained expressing in *E-coli.* The present invention discloses two recombinant lectins with modified amino acid sequences but have close resemblances with native lectin with respect to their binding properties towards cancer tissues, in vitro cultured cells, and their apoptotic activity to cultured cells. However these two lectins have better solubility and stability and desired sugar specificity properties for applications as compared to native lectin..

The recombinant lectin expressed as a soluble protein is purified by ion exchange and gel filtration chromatographic techniques.

The amino acid sequence SEQ ID 2 and 3 represents such lectin having all the above characteristics. The nucleic acid molecule encoding the amino acid for the protein of the above characteristics is also within the scope of the application. The DNA sequence of SEQ ID 1.1 and 2.1 represents such nucleic acid encoding the lectin having the above characteristics. The sequences also envisage any change in the nucleotide or amino-acid sequences including but not limited to any substitution, deletion, addition or modification such as acetylation, nitration, glycation , sulphonation etc. in any of the positions of the entire sequence and also to include truncations at 5' or 3' end of the sequences which do not alter the properties disclosed in the instant application.

In one aspect this recombinant lectin in a biotinylated form or modified with a fluorogenic chromophore can be used for the detection of cancer cells or cancer associated specific antigens.

The growth modulatory effect of the recombinant lectin can have potential application in cancer therapy as anti tumor agents.

In another aspect the recombinant lectin can be used in cancer therapy and research.

The application particularly discloses a DNA sequence (SEQ ID 1.1 and 2.1) coding for a 141 amino acids long lectin similar to the lectin (*S. rolfsii*), but with a few modifications was chemically synthesized and cloned into *E.coli.*

The method for producing said recombinant lectin has also been disclosed. The cloned lectin is expressed after induction with the inducer IPTG.

Specifically, the present invention provides a lectin protein comprising an amino acid sequence of SEQ ID 2 or 3. The lectin protein of the invention has better solubility and stability properties as compared to lectin of SEQ ID 1.

The present invention also provides the lectin protein of the present invention for use in cancer therapy and the use of the lectin protein of the present invention in *in* vitro detection of cancer cells.

The present invention also provides the lectin protein of the present invention for use in cancer therapy and the use of the lectin protein of the present invention in *in vitro* detection of cancer cells.

The present invention further provides a nucleic acid molecule encoding a lectin protein of the present invention, wherein said nucleic acid molecule is DNA or RNA, preferably wherein the nucleic acid molecule is SEQ ID 1.1 or 2.1.

The present invention also provides a recombinant vector comprising operatively linked in the 5' to 3' direction: a promoter which functions in a host cell; a structural nucleic acid sequence of the present invention wherein said nucleic acid sequence encodes a lectin protein; and a termination signal.

In addition, the present invention provides a transformed host cell containing the vector of the present invention.

Still further the present invention provides a process for producing a recombinant *Sclerotium rolfsii* lectin protein comprising: culturing a host cell containing the recombinant vector of the present invention coding for the lectin protein; expressing the recombinant lectin protein; and isolating said lectin protein from the culture.

### BRIEF DESCRIPTION OF FIGURES

**Figure 1****:** Comparative profiles of native SRL receptors isolated from the membranes of human normal resting PBMCs (1), PHA-activated PBMCs (2), Molt-4 (3) and Jurkat cells (4). Membrane proteins (40 µg) were resolved by SDS-PAGE (10% gel) and blotted on to nitrocellulose membrane. SRL interacting proteins were probed with biotinylated lectin and compared with standard molecular weight protein markers (M) after visualization with streptavidin-HRP reaction.

### Figure 2:

Binding pattern of Rec 2 and Rec 3 and native lectin to human cancerous tissues of breast and ovary in comparison with H & E staining. Biotinylated lectins were used for the immunohistochemistry

### Figure 3:

**The Amino acid sequence of lectin protein** isolated from *S. rolfsii* (SEQ ID 1); **Protein Sequence of the recombinant lectin Rec 2** of the instant application. The number of amino acid coding for this lectin is 141 and is represented by SEQ ID 2. The altered/modified positions have been highlighted in the sequence.

**Protein Sequence of the recombinant lectin Rec 3** of the instant application. The number of amino acid coding for this lectin is 141 and is represented by SEQ ID 3. The altered/modified positions have been highlighted in the sequence.

**Multiple sequence alignment** of the SEQ ID 1 -3.

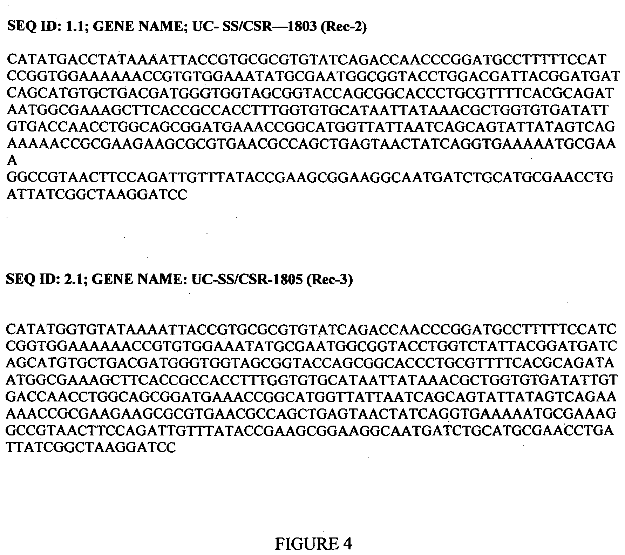

**Figure 4****: A DNA sequence encoding the lectin** of the instant application, SEQ ID 1.1 and 2.1 The length of the optimized sequence is ----- nucleotides which includes the 5' end Nde I (CATATG) and 3' end BamHI (GGATCC) restriction sites.SEQ ID: 1.1; GENE NAME; UC- SS/CSR-1803 (Rec-2); SEQ ID: 2.1; GENE NAME: UC-SS/CSR-1805 (Rec-3)

### DETAILED DESCRIPTION OF THE INVENTION

The present invention discloses cloned genes for two new variants, designated here as Rec-2 and Rec-3, in *E-coli* that are more close to the native *S. rolfsii* lectin in amino acid sequence, but still differ in surface charges. Surface charge was altered deliberately in order to enhance the better solubility and stability of the proteins as compared to native lectin. Two lectin genes coding *S. rolfsii* lectin with few alterations are chemically synthesized and expressed in *E-coli.* Recombinant lectins are purified by conventional chromatographic methods and are shown to have molecular mass approximate 16,000 Da. The two recombinant lectins described here are different in blood group specificity, recognize all the human blood groups and have exclusive ability to recognize TF antigen and its cryptic forms. The two blood group non specific recombinant lectins are more closely resemble with native *S*. *rolfsii* lectin in sugar binding, cell binding and in bringing apoptotic properties. However the recombinant lectins have better stability and solubility properties compared to native lectin making them ideal for various applications.

The various applications to the use of the recombinant lectins may be in the following:
Considering the property of cancer cell binding R1 and R2 can be used as drug delivery agents for cancer treatment and may also find application as diagnostic tools in the detection of microbial / viral pathogens.

These recombinant lectins can be used for the detection of certain glycoproteins and glycoconjugates present in biological fluids.

The lectins exhibits substrate specificity towards cancer associated cell surface carbohydrate such as desialylated TF antigen, they can have application as affinity matrices for the purification of glycoproteins and glycoconjugates of physiological and biochemical significance that bind to these recombinant lectins.

Recombinant lectins can be used as fusion partners for the purification of recombinant lectins.

The invention would now be disclosed by way of preferred methodology and embodiments:

### Lectin Gene construct and cloning:

The amino acid sequence derived from the MALDI MS/MS and x-ray crystallography data of the lectin purified from the soil fungus *Sclerotium rolfsii* formed the basis for the gene construct. The gene corresponding to this sequence SEQ ID 1 was chemically synthesized by following the standard protocols. The assembled gene was first inserted into a pUC57 vector. The insert was released by digesting the plasmid with NdeI & BamHI and then recloned in to pET20b previously digested NdeI and BamHI. After ligation the plasmid was used to transform *E.coli* DE3 (GOLD) host strain for expression. The recombinant clones were analyzed for release of insert after digestion with Nde I and Bam HI. The SDS-PAGE analysis of recombinant *E.coli* upon induction with IPTG showed the expected ∼16kDa protein.

### Cell growth

A single colony of the recombinant *E.coli* was inoculated into 5ml LB-Amp allowed to grow at 37°C overnight with shaking. The overnight grown culture was inoculated into the Fermentation Media and grown at 37°C till an OD of ∼2.0 was reached, the cultures were then induced with 250µM IPTG (final concentration) and grown overnight at 20°C.

### Cell extract preparation

The overnight grown cultures were centrifuged at 8000rpm for 10min and the pelleted cells (4gms) were suspended in 40ml of 50mM Tris-HCl pH8.0 containing 1mM PMSF and 1mM EDTA. The cell suspension was sonicated for 20min using the sonicator. The cells were centrifuged at 12000rpm, 10min at 4°C. The supernatant was used for further purification.

### Purification of the protein

### DEAE-cellulose chromatography:

The supernatant (400mg of protein) in 50 mM Tris-HCl buffer pH 8.0 was loaded on 25ml DEAE (BIORAD) column equilibrated in 50mM Tris-HCl buffer pH 8.0. The column was washed with 2 column volumes of buffer followed by step-wise elution of the column with buffer b containing 75mM NaCl and 200mM NaCl. The lectin was eluted with buffer containing 300mM NaCl. This fraction contained 216 mg of protein.

### PEI chromatography:

The DEAE elute (216mg) was loaded on to the NUCLEOSIL 4000-7-PEI (250 x 10mm) column, equilibrated with 50 mM Tris-acetate buffer pH 8.0. The bound protein was eluted by applying a linear gradient of NaCl from 0-100% in 15min at 2ml/min flow rate using the FPLC system and the protein eluted at 98%B (60mg). The eluted protein was dialyzed against water and repurified on the same PEI column using ammonium bicarbonate buffer.

The PEI column was equilibrated with 20mM ammonium bicarbonate buffer pH 8.0 and the protein was loaded on the column. The bound protein was eluted using buffer gradient from 20-500mM ammonium bicarbonate buffer. The eluted protein (30mg) was dialyzed against water, lyophilized and stored at -20°C.

Gel filtration chromatography: The final purification of the recombinant protein was achieved by gel filtration chromatography on Superdex G-75 equilibrated with 25 mM TBS, pH 7.2 on AKTA Prime plus purification system.

The expression of the protein and the purification of the protein were checked on 15% SDS PAGE and the results are given here. There was a major Coomassie blue stainable protein band corresponding to a molecular size of 16kDa.

### EXAMPLES

### Solubility studies:

Purified recombinant lectins were dialyzed extensively against water and lyophilized. Lyophyllized samples were suspended in buffers of different pH, the protein content and the hemagglutination activity were determined in the clear supernatant after centrifugation. Solubility and the hemagglutinating activities were compared with native lectin.

### Histochemical studies:

Binding of the lectins to tissue sections of confirmed cases of human colon epithelial cancer were performed using biotinylated lectins and the binding was observed by light microscopy. Tissue sections were stained routinely using hematoxylin and eosin and lectin histochemical studies were carried out by standard protocols using five µm tissue sections of cancerous tissues. Sections of colon cancer tissues each of 5 µm thickness used for histochemical studies were obtained using rotary microtome semi-automatic (Leica RM 2145) and deparaffinised by heating on a slide warmer at 60 °C for 1 h. The tissue sections were rehydrated by keeping them in two changes of xylene for five minutes each which were then transferred to absolute and 95% alcohol for three minutes each. As a routine procedure to confirm the state of tissues being in normal or cancerous condition, tissue sections were stained with hematoxylin and eosin (H and E) stains. Tissue sections, after rehydration were treated with basophilic hematoxylin stain for 20-25 min, followed by 1% HCl, acid-alcohol dip and the tissue sections were gently washed under running tap water. Then, sections were counterstained with eosin for 2 minutes, washed with water, dried and dipped in xylene and mounted with DPX (Distrene 80 Dibutyl Phthalate Xylene), a nonaqueous permanent mounting medium and observed using light microscope (Carl zeiss Jenalumar) and photographed.

Histochemical staining was carried out, using biotinylated lectins as described by Danguy and Gabius, in Gabius and Gabius (1993). All the reagents were equilibrated to room temperature prior to the staining. At no time the tissue sections were allowed to dry during the staining procedure. Study also included control slides prepared by excluding the biotinylated lectin. To block the endogenous peroxidase activity, tissue sections after rehydration were incubated with methanol containing 0.3% hydrogen peroxide for 30 minutes at room temperature. Sections were then rinsed with water for 5 minutes and then with TBST (pH 7.2) for 5 minutes. After the tissue sections were rinsed with PBS pH 7.2, sections were incubated with 1% periodate treated BSA in 0.1 M PBS solution for 15 min to block nonspecific binding sites by BSA. Sections were then rinsed with TBST for 5 min. Biotinylated lectins (20µg/ml in PBS) were carefully applied to cover the sections completely and incubated for 1 h, so as to facilitate the complete binding. Sections were then rinsed with TBST, for 5 minutes. These tissue sections were then incubated with streptavidin-HRP (1→1000 dilution) in PBS, pH 7.2, for 30 minutes at room temperature. Sections were then rinsed with two changes of TBST, pH 7.2, for 5 minutes. After streptavidin-HRP conjugation, tissue sections were developed in DAB Chromogen/H₂O₂ substrate in buffered solution (pH 7.5), which was prepared according to the guidelines of manufactures. Tissue sections were incubated for 10 minutes and then gently rinsed with water for 5 minutes. The slides were allowed to dry and dipped in xylene and mounted in DPX. Sections were then examined under light microscope (Carl zeiss Jenalumar) to observe and study the staining intensity patterns by the normal and cancer tissues and photographed.

### Identification of cell surface antigens:

Identification of neoglycans expressed on activated and transformed lymphocytes in comparison with normal lymphocytes by the recombinant lectin;

In order to identify the neoglycoproteins expressed on lymphocytes upon activation by PHA and transformed cells, the interaction studies were carried out with respective cell membrane proteins with biotinylated lectin.

### Isolation of Cell Membrane Proteins:

Human peripheral lymphocytes were prepared from the blood collected from a healthy donor. The blood sample (20ml) collected in to glass centrifuge tube containing EDTA (12.5 mM final concentration) and the normal lymphocytes were separated by centrifugation in Ficoll-Hypaque (Pharmacia) gradient. A portion of normal lymphocytes were activated by incubating with PHA (2.5 µg/ml PHA) in RPMI-1640 medium supplemented with 10% FCS for 72 hours in CO₂ incubator. Activated cells were harvested by centrifugation and the activation was confirmed by checking for the expression of CD25 receptors.

Normal lymphocytes, activated lymphocytes and leukemic cell lines; Molt-4 and Jurkat were finally maintained in RPMI 1640 culture medium, supplemented with glutamine (2 mM), heat inactivated FCS (10%), penicillin (100U/ml) and streptomycin (100-ug/ml). The cultures were maintained at 37°C in humidified atmosphere (95% air and 5% CO₂). Membrane proteins from these cultured cells were isolated by using "Pierce Membrane Protein Extraction Reagent kit" method (Mem-PER, Prod #89826) and the protein contents were estimated using Bio-Rad's DC-Protein estimation Kit. Isolated membrane proteins were finally concentrated by chloroform-methanol precipitation, and the precipitated proteins were air dried and stored at 4°C till further use.

### Electrophoresis & western Blotting:

Polyacrylamide gel electrophoresis was performed in the presence of SDS using a minigel system (Hoefer Scientific Instruments, USA) in 10% gel for 60 mins at 120 volts. The proteins were transferred from polyacrylamide gels to Nitro Cellulose membranes in a semidry blotting equipment using transfer buffer (240mM Glycine, 25mM Tris, 0.1% SDS) containing 20% methanol at 75mA for 3hr at 4°C. Membranes were saturated after transfer with blocking solution (3% p-BSA) and washed with TBST. Lectin binding was carried out at room temperature for 4 hours with biotin-coupled lectin at the final concentration of 20µg/ml (prepared in TBST). After thorough washing, blot was incubated with Streptavidin-HRP (1:1000 in TBST) at room temperature for 1hr and excess Streptavidin-HRP was washed with TBST. Finally the lectin-glycoproteins bands were visualized by developing with DAB chromogenic system.

The results of these findings show that the present recombinant lectin has the ability to bind to some of the uniquely expressed neoglycans on the leukemic cells and also activated lymphocytes.

### SDS PAGE and lectin blotting

Proteins from the ovarian cyst fluids samples were separated by SDS PAGE in 10 % gels as described by Laemmli et al using Hoefer Scientific Instruments, San Francisco, USA. Electrophoresis was carried out for 70 min. using 110 volts. Immediately after the electrophoresis fractionated protein bands were resolved were blotted onto nitro cellulose membrane using semidry blot assembly. Blotted membrane was washed with distilled water and stained with Ponceau reagent to confirm the efficiency of blotting and to mark the location of standard proteins. Finally the blot was washed with TBS containing 0.1 % Tween-20 (TBST) and treated with 3 % P-BSA in TBST for overnight to prevent non-specific binding. After washing with TBST, blot was incubated with biotinylated lectins (20 µg/ ml in TBST) at 37°C for 4-6 hours. Excess and unbound lectin was removed by washing the membrane with TBST and then the membranes were incubated with Streptavidin-HRP (1µg/ml in TBST) for one hour. Unbound Streptavidin-HRP was removed by washing with TBST. The lectin binding glycoproteins were visualized by staining for peroxidase activity using DAB system.

### Proliferative and Antiproliferative activity on PBMCs and cultured leukemic and colon cancer cells

Proliferative/antiproliferative activity of SRL on human peripheral blood mononuclear cells (PBMCs) was determined by tritiated thymidine incorporation assay, using freshly isolated human PBMCs as described by Wang et al., [1992] with some modifications. PBMCs were isolated from the freshly collected blood samples of healthy donors and the cells were maintained in RPMI 1640 complete medium as described previously. The cells were diluted with RPMI medium containing 10% FCS and then seeded (105 cells/50 µl/well) in 96-well microplates (NUNC Denmark). Various concentrations (10, 5, 2.5, 1.25 and 0.625 µg/ml) of SRL in RPMI 1640 medium was added to each well in a final volume of 100 µl and incubated at 37°C in a humidified, 5% CO₂ atmosphere for 72 h. Eighteen hours prior to the total incubation period, 1 µCi of tritiated thymidine was added to each well in a final volume of 10 µl and the incubation was continued for another 18 h. Finally, cells were harvested onto a glass filter paper (Amersham Biosciences) using cell harvester (NUNC, Denmark), washed thoroughly with distilled water and dried in micro-oven. Filter discs were transferred to scintillation vials (Tarsons, India) containing 600 µl of toluene-based scintillation cocktail. Finally the incorporated tritiated thymidine was measured by β-scintillation counter (Packard Bioscience Ltd.) as counts per minute (CPM). In order to confirm the lectin-receptor mediated proliferative activity, assays were performed using SRL preincubated with mucin. SRL (10 µg/ml) was preincubated with mucin (12.5 µg in 100 µl) for 1 h at 37°C and used for cell proliferation assay. Appropriate controls containing cells without lectin and cells with mucin alone were included. Results are representative of three independent experiments done in triplicate.

Proliferative/antiproliferative effect of SRL on Molt-4 leukemic cell line was determined by tritiated thymidine incorporation assay as described previously. Proliferative/antiproliferative effect of SRL on Molt-4 leukemic cell line was determined by tritiated thymidine incorporation assay as described previously. Cells were seeded (105 / well) in a 96-well tissue culture plate and allowed to acclimatize for 3h before lectin treatment Serial concentrations of SRL from 6.25 µg/ml to 100 µg/ml in 50 µl of RPMI medium was added to each well and incubated for 72 h. All other steps of assay remained same as described earlier. For lectin blocking assay, SRL (100 µg/ml) was preincubated with 12.5 µg of mucin in a total volume of 100 µl for 1 h at 37°C and used for assay.

### Recombinant Sequences

The gene sequences and the proteins having the following biological activity: recognize all the human blood groups and have exclusive ability to recognize TF antigen and its cryptic forms, resemble with native *S. rolfsii* lectin in sugar binding, cell binding and in bringing apoptotic properties, have better stability and solubility properties compared to native lectin
are covered within the scope of the application. The nucleotide molecules encoding the recombinant molecules may be DNA or RNA. The proteins of the above mentioned characteristics whether obtained by deliberate or random mutations are covered within the concept of the instant application.

Binding of recombinant lectins to different cultured cells are presented in table 1.

**Table; 1, Binding of Recombinant lectins to Cultured leukemic (Molt 4& Jurkat), Ovarian (PA 1) and colon (COLO 205) cells and the inhibition of binding by mucin**

| **Cell lines** | | **% positive cells** | | |
|---|---|---|---|---|
| | | Rec 2 | Rec 3 | Native |
| Molt 4 | Lectin | 84.03 | 99.87 | 88.45 |
| | Lectin+Mucin | 0.24 | 0.30 | 0.24 |
| Jurkat | Lectin | 81.35 | 97.85 | 33.84 |
| | Lectin+Mucin | 0.89 | 1.02 | 0.50 |
| PA-1 | Lectin | 97.97 | 97.58 | 96.72 |
| | Lectin+Mucin | 2.5 | 4.28 | 1.63 |
| COLO 205 | Lectin | 66.53 | 90.29 | 91.66 |
| | Lectin+Mucin | 0.87 | 1.08 | 0.99 |

In each case binding of R2 and R3 with Molt 4, Jurkat (leukemic cells), PA-1 (ovarian cells) and COLO 205 (colon cells) is greater than 90 % and the binding can be abolished by mucin.

As another embodiment the immunomodulatory effect on these cultured cells is disclosed. Both R2 and R3 exerted anti proliferative activity on the cultured cells, highest effect (60%) on leukemic cells as compared to ovarian cells (50%) and colon cells (40%). their growth modulatory effect can have potential application in cancer therapy. (table 2)

**Table; 2, Anti proliferative activity of recombinant lectins on cultured cells by MTT assay**

| **Cell lines** | **Decrease in cell viability (%)** | | |
|---|---|---|---|
| | Rec 2 (25 µg/ml) | Rec 3 (25 µg/ml) | Native (25 µg/ml) |
| Molt 4 | 60.68 | 61.10 | 57.93 |
| PA-1 | 46.56 | 50.60 | 53.85 |
| COLO-205 | 26.67 | 40.43 | 23.44 |

In one of the preferred embodiments, the expressed lectin protein (SEQ ID 2 and 3) differs from the native lectin protein in terms of at least one amino acid modification/substitution at positions. Of these, positions changes are indicated in Table 3

**Table: 3: Amino acid positions changed for Rec 2 and Rec 3 compared to native lectin sequence**

| **Lectin** | **Amino acid positions changed** | | | | |
|---|---|---|---|---|---|
| | **1** | **14** | **34** | **113** | **123** |
| Native | Ac-T | N | T | E | E |
| S1 | T | D | T | Q | Q |
| S2 | V | D | s | Q | Q |

The recombinant lectin sequences can be biotinylated or attached with fluorogenic chromophore by using commonly known methods to be used for the detection of cancer cells or cancer associated specific antigens. The growth modulatory effect of the recombinant lectin finds its application in cancer therapy. Further the use of recombinant lectin as a drug delivery agent for cancer treatment is envisaged in the present application.

## Claims

1. A lectin protein comprising an amino acid sequence of SEQ ID 2 or 3, which has better solubility and stability properties as compared to a lectin protein of SEQ ID 1.

2. The lectin protein as claimed in claim 1 wherein said lectin has a molecular weight of approximate 16.1 kDa and PI value of 6.614.

3. The lectin protein as defined in claim 1 or 2 for use in cancer therapy.

4. Use of a lectin protein as defined in claim 1 or 2 in *in vitro* detection of cancer cells.

5. A nucleic acid molecule encoding a lectin protein as defined in claim 1 or 2, wherein said nucleic acid molecule is DNA or RNA.

6. A nucleic acid molecule as defined in claim 5, wherein said nucleic acid molecule is DNA of SEQ ID 1.1 or 2.1.

7. A recombinant vector comprising operatively linked in the 5' to 3' direction: a promoter which functions in a host cell; a structural nucleic acid sequence as defined in claim 5 or 6 wherein said nucleic acid sequence encodes a lectin protein; and a termination signal.

8. A recombinant vector as claimed in claim 7 wherein said recombinant vector is capable of being replicated, transcribed, translated and expressed in a unicellular organism.

9. A transformed host cell containing the vector as claimed in claim 7 or 8.

10. The transformed host cell as claimed in claim 9 wherein said host cell is *Escherichia coli* bacterium or yeast cell.

11. A process for producing a recombinant *Sclerotium rolfsii* lectin protein comprising:
• culturing a host cell containing the recombinant vector as defined in claim 7 or 8 coding for the lectin protein;
• expressing the recombinant lectin protein; and
• isolating said lectin protein from the culture.

## Patentansprüche

1. Lektinprotein, umfassend eine Aminosäuresequenz von SEQ ID 2 oder 3, das bessere Löslichkeits- und Stabilitätseigenschaften im Vergleich zu einem Lektinprotein von SEQ ID 1 hat.

2. Lektinprotein nach Anspruch 1, wobei das Lektin ein Molekulargewicht von ungefähr 16,1 kDa und einen PI-Wert von 6,614 hat.

3. Lektinprotein nach Anspruch 1 oder 2 zur Verwendung in der Krebstherapie.

4. Verwendung eines Lektinproteins nach Anspruch 1 oder 2 in der *In-vitro*-Erkennung von Krebszellen.

5. Nukleinsäuremolekül, das für ein Lektinprotein nach Anspruch 1 oder 2 codiert, wobei das Nukleinsäuremolekül DNA oder RNA ist.

6. Nukleinsäuremolekül nach Anspruch 5, wobei das Nukleinsäuremolekül DNA von SEQ ID 1.1 oder 2.1 ist.

7. Rekombinanter Vektor, umfassend funktionell verbunden in der 5'- zu 3'-Richtung: einen Promotor, der in einer Wirtszelle wirkt; eine strukturelle Nukleinsäuresequenz nach Anspruch 5 oder 6, wobei die Nukleinsäuresequenz für ein Lektinprotein codiert; und ein Beendigungssignal.

8. Rekombinanter Vektor nach Anspruch 7, wobei der rekombinante Vektor in der Lage ist, in einem einzelligen Organismus repliziert, transkribiert, übersetzt und exprimiert zu werden.

9. Transformierte Wirtszelle, die den Vektor nach Anspruch 7 oder 8 enthält.

10. Transformierte Wirtszelle nach Anspruch 9, wobei die Wirtszelle ein *Escherichia coli*-Bakterium oder eine Hefezelle ist.

11. Verfahren zum Produzieren eines rekombinanten *Sclerotium rolfsii-*Lektinproteins, umfassend:
• Kultivieren einer Wirtszelle, die den rekombinanten Vektor nach Anspruch 7 oder 8 codierend für das Lektinprotein enthält;
• Exprimieren des rekombinanten Lektinproteins; und
• Isolieren des Lektinproteins aus der Kultur.

## Revendications

1. Protéine lectine comprenant une séquence d'acides aminés de la SEQ ID n° : 2 ou 3, qui présente de meilleures propriétés de solubilité et stabilité par rapport une protéine lectine de la SEQ ID n° : 1.

2. Protéine lectine telle que définie dans la revendication 1, ladite lectine présentant un poids moléculaire d'environ 16,1 kDa et une valeur PI de 6,614.

3. Protéine lectine telle que définie dans la revendication 1 ou 2 destinée à une utilisation dans un traitement du cancer.

4. Utilisation d'une protéine lectine telle que définie dans la revendication 1 ou 2 dans une détection *in vitro* de cellules cancéreuses.

5. Molécule d'acide nucléique codant pour une protéine lectine telle que définie dans la revendication 1 ou 2, ladite molécule d'acide nucléique étant l'ADN ou l'ARN.

6. Molécule d'acide nucléique telle que définie dans la revendication 5, ladite molécule d'acide nucléique étant l'ADN de la SEQ ID n° : 1.1 ou 2.1.

7. Vecteur recombinant comprenant, liés de manière fonctionnelle, dans la direction 5' à 3' : un promoteur qui fonctionne dans une cellule hôte ; une séquence d'acide nucléique structurel telle que définie dans la revendication 5 ou 6, ladite séquence d'acide nucléique codant pour une protéine lectine ; et un signal de terminaison.

8. Vecteur recombinant tel que défini dans la revendication 7, ledit vecteur recombinant étant capable d'être répliqué, transcrit et exprimé dans un organisme unicellulaire.

9. Cellule hôte transformée contenant le vecteur tel que défini dans la revendication 7 ou 8.

10. Cellule hôte transformée telle que définie dans la revendication 9, ladite cellule hôte étant la bactérie *Escherichia coli* ou une cellule de levure.

11. Processus de production d'une protéine lectine *Sclerotium rolfsii* recombinante comprenant :
• la mise en culture d'une cellule hôte contenant le vecteur recombinant tel que défini dans la revendication 7 ou 8 codant pour la protéine lectine ;
• l'expression de la protéine lectine recombinante ; et
• l'isolation de ladite protéine lectine de la culture.
